# EUROPÄISCHE PATENTSCHRIFT

(11) **EP 3 600 228 B1**
(45) Veröffentlichungstag und Bekanntmachung des Hinweises auf die Patenterteilung: **30.09.2020**
(21) Anmeldenummer: 18711513.4
(22) Anmeldetag: 12.03.2018
(51) Int. Cl.: A61K 8/31, A61K 8/34, A61K 8/36, A61K 8/37, A61K 8/68, A61K 8/06, A61K 8/02, A61Q 19/00

(54) **VERFAHREN UND ERZEUGNIS ZUR HERSTELLUNG CERAMIDHALTIGER FORMULIERUNGEN**
METHOD AND PRODUCT FOR PRODUCING FORMULATIONS CONTAINING CERAMIDE
PROCÉDÉ ET PRODUIT DE FABRICATION DE FORMULATIONS À BASE DE CÉRAMIDE

(30) Priorität: 27.03.2017 EP 17163002
(43) Veröffentlichungstag der Anmeldung: 05.02.2020
(73) Patentinhaber: Evonik Operations GmbH, 45128 Essen (DE)
(72) Erfinder: MACZKIEWITZ, Ursula, 45219 Essen (DE); MECKING, Maria, 46244 Bottrop (DE); SCHRADER, Annika, 28209 Bremen (DE)
(74) Vertreter: Evonik Patent Association
(86) Internationale Anmeldenummer: PCT/EP2018/056032
(87) Internationale Veröffentlichungsnummer: WO 2018/177730

(56) Entgegenhaltungen:
- EP-A1- 1 426 401
- EP-A1- 1 955 692
- EP-A2- 0 797 976
- WO-A2-2011/114214
- FR-A1- 2 759 902
- US-A1- 2004 005 282
- LEE TU ET AL: "Bio-inspired phase change materials designed for high specific heat of solid phase", THERMOCHIMICA ACTA, ELSEVIER SCIENCE PUBLISHERS, AMSTERDAM, NL, Bd. 591, 31. Juli 2014 (2014-07-31), Seiten 61-67, XP029045099, ISSN: 0040-6031, DOI: 10.1016/J.TCA.2014.06.009

## Beschreibung

### Gebiet der Erfindung

Gegenstand der Erfindung ist ein Verfahren zu Herstellung einer ceramidhaltigen Formulierung umfassend die Verfahrensschritte
A) Aufschmelzen einer Mischung enthaltend mindestens ein Wachs und mindestens ein Ceramid,
B) Abkühlen der aufgeschmolzenen Mischung unter Erhalt einer erstarrten Schmelze enthaltend das mindestens eine Wachs und das mindestens eine Ceramid,
C) Einarbeiten der erstarrten Schmelze in eine ölhaltige Formulierung.

### Stand der Technik

Ceramide werden vielfach in kosmetische Formulierungen eingesetzt. Ihre geringe Löslichkeit und große Neigung zu Rekristallisation erschweren jedoch die stabile Einarbeitung in kosmetischen Formulierungen.
Der Stand der Technik adressiert dieses Problem durch verschiedene Darreichungsformen.

So beschreibt die WO1998053797 eingekapselte, wasserunlösliche Wirkstoffe mit amphiphilem Charakter, mit einem Gehalt an Wasser und mindestens einem Tensid aus der Gruppe der Ester von langkettigen Carbonsäuren mit Hydroxylgruppen enthaltenden Carbonsäuren oder deren Salzen und der Ester von langkettigen Carbonsäuren mit Polyalkoholen, wobei Ceramide die wasserunlöslichen Wirkstoffe sein können.

Die WO1999029293 beschreibt Zusammensetzung zur topischen Anwendung, enthaltend eine Kombination einer freien Sphingoidbase und eines Ceramids.

Die JP2008297301 beschreibt Ceramide und Phytostyrol-Derivate in triglyceridhaltigen Öl-Lösungen in flüssiger Form zur Einarbeitung in dermatologische Zubereitungen.

Aufgabe der Erfindung war es, eine Möglichkeit der Einarbeitung von Ceramiden in Formulierungen, insbesondere in Öl-in-Wasser-Emulsionen, bereitzustellen, die die oben beschriebenen Probleme des Standes der Technik löst.

### Beschreibung der Erfindung

Überraschenderweise wurde gefunden, dass das im Folgenden beschriebene Verfahren die der Erfindung gestellte Aufgabe zu lösen vermag.

Gegenstand der vorliegenden Erfindung ist daher ein Verfahren zu Herstellung einer ceramidhaltigen Formulierung wie in Anspruch 1 beschrieben.
Ein weiterer Gegenstand der Erfindung ist eine erstarrte Schmelze enthaltend mindestens ein Wachs und mindestens ein Ceramid.

Ein Vorteil der vorliegenden Erfindung ist es, dass die Löslichkeitstemperatur für die Ceramide in Ölen erniedrigt wird. Dadurch ist es möglich, dass man energiesparend ceramidhaltige Formulierungen herstellen kann, da man nur eine geringere Substanzmenge zu hohen Temperaturen erwärmen muss, und der verbleibende Großteil der Formulierungsbestandteile bei niederen Temperaturen formuliert werden kann.
Hierdurch entsteht ein weiterer Vorteil, nämlich dass temperaturempfindliche Inhaltsstoffe in ceramidhaltige Formulierungen eingearbeitet werden können.
Oft stellt die hohe Löslichkeitstemperatur der Ceramide in Ölen ein technisches Problem dar, da z.B. mit Wasserdampfheizungen ausreichend hohe Temperaturen nicht erreicht werden. Auch dieses Problem wird durch die Erfindung gelöst.
Ein weiterer Vorteil der vorliegenden Erfindung ist es, dass Ceramide aufgrund der vorliegenden Erfindung auch im Eintopf-Verfahren in Formulierungen eingebracht werden können.
Noch ein Vorteil der vorliegenden Erfindung ist es, dass die erfindungsgemäßen Formulierungen eine erhöhte Lagerstabiliät aufweisen, daher sich langsamer in ihrer Beschaffenheit, insbesondere hinsichtlich ihrer Viskosität, verglichen zu ceramidhaltigen Formulierungen nach dem Stand der Technik über die Zeit verändern.
Noch ein Vorteil der vorliegenden Erfindung ist es, dass die erfindungsgemäßen Formulierungen eine höhere Anzahl von Gefrier-Tauschritten ohne signifikante Viskositätseinbuße vertragen, verglichen zu ceramidhaltigen Formulierungen nach dem Stand der Technik.

Gegenstand der Erfindung ist ein Verfahren zu Herstellung einer ceramidhaltigen Formulierung umfassend die Verfahrensschritte
A) Aufschmelzen einer Mischung enthaltend mindestens ein Wachs und mindestens ein Ceramid,
B) Abkühlen der aufgeschmolzenen Mischung unter Erhalt einer erstarrten Schmelze enthaltend das mindestens eine Wachs und das mindestens eine Ceramid,
C) Einarbeiten der erstarrten Schmelze in eine ölhaltige Formulierung.

Unter dem Begriff "Wachs" im Zusammenhang mit der vorliegenden Erfindung werden Substanzen verstanden, welche bei 20 °C und 1 bar fest sowie durchscheinend bis opak, jedoch nicht glasartig, sind und bei 1 bar einen Schmelzpunkt von 40 °C oder höher aufweisen.
Unter dem Begriff "Ceramid" im Zusammenhang mit der vorliegenden Erfindung werden acylierte Sphingoidbasen verstanden, wobei die Sphingoidbasen ausgewählt sind aus Sphingosin, Sphinganin, 6-Hydroxysphingosin und Phytosphingosin.
Alle angegebenen Prozent (%) sind, wenn nicht anders angegeben, Massenprozent.

Es ist erfindungsgemäß bevorzugt, wenn die in Verfahrensschritt A) eingesetzten Wachse kosmetische Wachse sind; diese sind insbesondere ausgewählt aus der Gruppe bestehend aus natürliche Wachse pflanzlichen oder tierischen Ursprungs, Mineralwachse, Fette, Fettalkohole, Fettsäure, Fettsäureester, Ether von Fettalkoholen, Ester aus Polyethylenoxid und Fettsäuren, Ether aus Polyethylenoxid und Fettalkoholen und partielle Glycerinester.
Bevorzugt natürliche Wachse pflanzlichen oder tierischen Ursprungs sind ausgewählt aus der Gruppe bestehend aus Baumwollwachs, Carnaubawachs, Candelillawachs, Espartowachs, Guarumawachs, Japanwachs, Korkwachs, Montanwachs, Ouricurywachs, Reiskeimölwachs, Zuckerrohrwachs, Bienenwachs, Bürzeldrüsenfett, Wollwachs, Schellackwachs, Walrat, hydriertes Ricinusöl und Shea Butter.
Bevorzugte Mineralwachse sind ausgewählt aus der Gruppe bestehend aus Mikrowachse, Ceresin, Petrolatum und Ozokerit.
Bevorzugte Fettalkohole sind ausgewählt aus der Gruppe bestehend aus gesättigten Fettalkoholen mit einer Kohlenstoffkettenlänge von 10 bis 22 C-Atomen.
Bevorzugte Fettsäuren sind ausgewählt aus der Gruppe bestehend aus Fettsäuren mit einer Kohlenstoffkettenlänge von 10 bis 22 C-Atomen.
Bevorzugte Fettsäureester sind ausgewählt aus der Gruppe bestehend aus Ester von Fettalkoholen, Ester von Glycerin und Estern von Polygylcerin, Ester von Polyethyleneoxid, Ester von Sorbitan, Ester von Zuckern wie z.B. Glucose oder Saccharose, insbesondere Glycerinstearate, Polyglyceryl-3 Methylglucose Distearate, Polyglyceryl-3 Dicitrate/Stearate, Glyceryl Stearate Citrate

Es ist erfindungsgemäß bevorzugt, wenn das in Verfahrensschritt A) eingesetzte Ceramid ausgewählt ist aus der Gruppe umfassend, bevorzugt bestehend aus, Ceramid NP, Ceramid AP, Ceramid EOP, Ceramid NDS , Ceramid ADS, Ceramid EODS, Ceramid NS, Ceramid AS, Ceramid EOS, Ceramid NH, Ceramid AH und Ceramid EOH, wobei Ceramid NP, Ceramid AP, Ceramid EOP, Ceramid NDS , Ceramid ADS, Ceramid EODS, Ceramid NS, Ceramid AS, Ceramid EOS bevorzugt ist, und wobei Ceramid NP besonders bevorzugt ist.

Das Gewichtsverhältnis von Wachs zu Ceramid in Verfahrensschritt A) sowie in der erstarrte Schmelze in Verfahrensschritt B) beträgt erfindungsgemäß bevorzugt 50 : 1 bis 1 : 1, bevorzugt 20 : 1 bis 2 : 1, besonders bevorzugt 9 : 1 bis 4 : 1.

Ein erfindungsgemäß bevorzugtes Verfahren ist dadurch gekennzeichnet, dass die Mischung in Verfahrensschritt A) und die erstarrte Schmelze in Verfahrensschritt B) bezogen auf die Gesamtmischung beziehungsweise Gesamtschmelze 70 Gew.-% bis 100 Gew.-%, bevorzugt 81 Gew.-% bis 99 Gew.-%, besonders bevorzugt 95 Gew.-% bis 98 Gew.-%, das mindestens eine Wachs und das mindestens eine Ceramid enthalten.

Die Mischung wird in Verfahrensschritt A) erfindungsgemäß bevorzugt bei einer Temperatur von 80 °C bis 105 °C, bevorzugt von 85 °C bis 99 °C, besonders bevorzugt von 87 °C bis 92 °C, aufgeschmolzen.

Ein erfindungsgemäß bevorzugtes Verfahren ist dadurch gekennzeichnet, dass die ölhaltige Formulierung in Verfahrensschritt C) eine Emulsion, insbesondere eine Öl-in-Wasser-Emulsion, ist und die Verfahrensschritte umfasst
C1) Erwärmen einer Öl-Phase enthaltend die erstarrte Schmelze,
C2) Erwärmen einer wässrigen Phase
C3) Homogenisieren der miteinander vereinigten Öl-Phase und wässrigen Phase.

Es ist erfindungsgemäß bevorzugt, dass in Verfahrensschritt C1) und C2) auf eine Temperatur von 60 °C bis 89 °C, bevorzugt von 65 °C bis 79 °C, besonders bevorzugt von 69 °C bis 76 °C, erwärmt wird.

Es ist erfindungsgemäß bevorzugt, dass in Verfahrensschritt C3) die Öl-Phase zur wässrigen Phase gegeben wird, wobei die Zugabe erfindungsgemäß bevorzugt unter Rühren erfolgt. Alternativ erfindungsgemäß bevorzugt kann in Verfahrensschritt C3) die wässrige Phase zur Öl-Phase gegeben werden, wobei die Zugabe erfindungsgemäß bevorzugt unter Ausschluss von Rühren erfolgt.

Es ist vorteilhaft und daher erfindungsgemäß bevorzugt, dass in der Öl-Phase mindestens ein Öl und mindestens ein Emulgator, insbesondere ein Öl-in-Wasser-Emulgator, enthalten sind.

Erfindungsgemäß bevorzugt enthaltene Öle sind kosmetische Öle. Unter dem Begriff "kosmetisches Öl" im Zusammenhang mit der vorliegenden Erfindung sind mit Wasser nichtmischbare Flüssigkeiten (d.h. bei 25 °C und 1 bar flüssige), welche zur Herstellung kosmetischer Formulierungen geeignet sind, zu verstehen. Mit Wasser nicht mischbar bedeutet im Zusammenhang mit der vorliegenden Erfindung, dass bei Raumtemperatur wässrige Mischungen von kosmetischen Ölen bei Ölkonzentrationen von 0,5 - 99,5 vol.-% bezogen auf die Gesamtmischung zu einer bereits mit dem menschlichen Auge wahrnehmbaren Trübung bzw. zur Ausbildung von zwei oder mehr Phasen führen. Des Weiteren sind im Zusammenhang mit der vorliegenden Erfindung kosmetische Öle bevorzugt dadurch gekennzeichnet, dass sie zu Wasser ein Grenzflächenspannung von > 5 mN/m aufweisen. Kosmetische Öle können zum Beispiel Oleochemie- oder Silikonchemie basiert sein.
Insbesondere sind diese ausgewählt aus der Gruppe der Fettalkohole, Ester von linearen Fettsäuren mit linearen oder verzweigten Fettalkoholen, Ester von verzweigten Fettsäuren mit linearen oder verzweigten Fettalkoholen, Ester von linearen Fettsäuren mit unverzweigten oder verzweigten mehrwertigen Alkoholen, Ester von verzweigten Fettsäuren mit unverzweigten oder verzweigten mehrwertigen Alkoholen, Ester von linearen Fettsäuren mit unverzweigten oder verzweigten Alkoholen, Ester von verzweigten Fettsäuren mit unverzweigten oder verzweigten Alkoholen, Ester von Alkylhydroxycarbonsäuren mit linearen oder verzweigten Fettalkoholen. Des Weiteren Mono-, Di- oder Triglyceride in flüssiger Form. Des Weiteren Ester von Carbonsäuren, aromatischen Carbonsäuren oder Dicarbonsäuren mit linearen oder verzweigten Fettalkoholen, unverzweigten oder verzweigten mehrwertigen Alkoholen oder unverzweigten oder verzweigten Alkoholen. Des Weiteren lineare, cyclische oder verzweigte Kohlenwasserstoffe, mit oder ohne Substituenten, mit oder ohne Doppelbindungen. Des Weiteren pflanzliche Öle, Carbonate mit unverzweigten oder verzweigten Alkoholen, Carbonate mit unverzweigten oder verzweigten mehrwertigen Alkoholen, Carbonate mit linearen oder verzweigten Fettalkoholen. Des Weiteren Ether, mit oder ohne Alkoxygruppen, Silikonöle, mit oder ohne organische Modifizierung. Des Weiteren Mischungen dieser Öle in beliebigen Verhältnissen.

Erfindungsgemäß bevorzugt in der Öl-Phase enthaltene Öl-in-Wasser-Emulgatoren sind ausgewählt aus der Gruppe bestehend aus Ester von Fettsäuren mit Glycerin, Polyglycerin, Polyethylenoxid, Zuckern oder Sorbitan, Fettsäuresalze, ethoxylierte Fettalkohole, ethoxylierte Fettsäuren und ethoxylierte Silikone.
Bevorzugte als Öl-in-Wasser-Emulgatoren in der Öl-Phase enthaltene Ester von Fettsäuren sind ausgewählt aus der Gruppe bestehend aus Polyglyceryl-3 Dicitrate/Stearate, Polyglyceryl-4 Laurate, Methylglucose Sesquistearate.
Bevorzugte als Öl-in-Wasser-Emulgatoren in der Öl-Phase enthaltene ethoxylierte Fettalkohole und ethoxylierte Fettsäuren sind ausgewählt aus der Gruppe bestehend aus Ceteareth-25, Ceteareth-15, PEG-40 Stearate.
Bevorzugte als Öl-in-Wasser-Emulgatoren in der Öl-Phase enthaltene ethoxylierte Silikone sind ausgewählt aus der Gruppe bestehend aus Bis-PEG/PPG-20/5 PEG/PPG Dimethicone, Methoxy PEG/PPG-25/4 Dimethicone, Bis-PEG/PPG-16/16 PEG/PPG-16/16 Dimethicone.
Der bevorzugt in der Öl-Phase enthaltene Öl-in-Wasser-Emulgator kann gegebenenfalls bezüglich seiner chemischen Zusammensetzung zu den erfindungsgemäß eingesetzten Wachsen identisch sein, liegt jedoch zu Beginn des Verfahrensschrittes C) in der Öl-Phase gelöst vor, und lässt sich somit von den in der erstarrten Schmelze enthaltenen Wachsen, die zu diesem Zeitpunkt als Feststoff vorliegen, eindeutig abgrenzen.

Das Gewichtsverhältnis von Öl-Phase zu wässriger Phase in Verfahrensschritt C3) beträgt erfindungsgemäß bevorzugt 1 : 100 bis 1: 1, bevorzugt 1: 50 bis 1 : 1,5, besonders bevorzugt 1 : 10 bis 1 : 2.

Ein weiterer Gegenstand der vorliegenden Erfindung ist das Zwischenprodukt des erfindungsgemäßen Verfahrens, eine erstarrte Schmelze einer Mischung enthaltend mindestens ein Wachs und mindestens ein Ceramid, dadurch charakterisiert, dass bezogen auf die gesamte erstarrte Schmelze Wachs und Ceramid in einer Menge von mindestens 80 Gew.% in ihr enthalten sind. Aus dem Wortlaut geht klar und eindeutig hervor, dass unter dem vorgenannten Begriff eine erstarrte Schmelze gemeint ist, die sich aus von einer zuvor aufgeschmolzenen Mischung mindestens eines Wachses und mindestens eines Ceramids ergibt.
Die erfindungsgemäßen erstarrten Schmelzen einer Mischung enthaltend mindestens ein Wachs und mindestens ein Ceramid sind bevorzugt dadurch charakterisiert, dass bezogen auf die gesamte erstarrte Schmelze Wachs und Ceramid in einer Menge von mindestens 90 Gew.-%, besonders bevorzugt mindestens 98 Gew.-% in ihr enthalten sind.
Insbesondere bevorzugt enthalten die erfindungsgemäßen erstarrten Schmelzen das Ceramid bezogen auf die gesamte erstarrte Schmelze in einer Menge von 2 Gew.-% bis 19 Gew.-%, bevorzugt von 5 Gew.-% bis 18 Gew.-%, besonders bevorzugt von 12 Gew.-% bis 17 Gew.-% enthalten ist.

In erfindungsgemäß bevorzugten erstarrten Schmelzen sind die in erfindungsgemäß bevorzugten Verfahren bevorzugt eingesetzten Wachse und Ceramide sowie bevorzugte Kombinationen der beiden Komponenten enthalten.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### Beispiel 1: Herstellung von erstarrten Schmelzen enthaltend Ceramid NP und Wachse

1 g Ceramid NP wurde jeweils zusammen mit 5 g Glycerinstearat (TEGIN M® Pellets, Evonik Industries), Stearinsäure, Polyglyceryl-3 Dicitrate/Stearate (TEGO® Care PSC 3, Evonik Industries) oder Cetearyllalkohol (TEGO® Alkanol 1618®, Evonik Industries) auf 90 °C unter Rühren erwärmt, bis eine homogene Flüssigkeit entstand. Anschließend wurde auf Umgebungstemperatur (ca. 20 °C) erkalten lassen, wodurch eine feste Masse entstand. Diese wurde in Pellets zerkleinert.

### Beispiel 2: Solubilisierungstemperatur-Erniedrigung in Ölen

Es wurde die minimal erforderliche Solubilisierungstemperatur für 0,1 g Ceramid NP in 20 g verschiedener Öle ermittelt.
Die Ergebnisse sind in Tabelle 1 dargestellt und zeigen, dass sich die minimal erforderliche Solubilisierungstemperatur signifikant reduzieren lässt, wenn Ceramid NP in Form einer erstarrten, wachshaltigen Schmelze solubilisiert wird.

**Tabelle 1: erforderliche Solubilisierungstemperaturen in °C**

| | Ceramid NP | Ceramid NP / TEGIN® M | Ceramid NP / TEGO® Alkanol 1618 | Ceramid NP / Stearic Acid |
|---|---|---|---|---|
| Persea gratissima (Avocado) Oil | 100 | 85 | 85 | 90 |
| Caprylic/Capric Triglycerides (TEGOSOFT® CT) | 95 | 75-80 | 85 | 80 |
| Ethylhexyl Stearate (TEGOSOFT® OS) | 100 | 85 | 85-90 | 85 |

### Beispiel 3: Formulierungstemperatur-Erniedrigung in O/W-Emulsionen

Die Formulierungen O/W Face Cream der Tabelle 2 wurden wie folgt hergestellt:
Phase A und B wurden separat auf die angegeben Temperaturen erhitzt, Phase A zu Phase B unter Rühren hinzugefügt und homogenisiert. Es wurde unter langsamem Rühren erkalten lassen.

**Tabelle 2**

| **Phase** | **Inhaltsstoff** | **w/w%** | **w/w%** | **w/w%** |
|---|---|---|---|---|
| | | VF1 | EF1 | EF2 |
| A | Polyglyceryl-3 Methylglucose Distearate (TEGO® Care 450) | 3.0 | 3.0 | 3.0 |
| | Glyceryl Stearate (TEGIN® M Pellets) | 2.0 | 1.5 | 2.0 |
| | Cetearyl Alcohol (TEGO® Alkanol 1618) | 1.0 | 1.0 | 0.5 |
| | Caprylic/Capric Triglyceride (TEGOSOFT® CT) | 6.0 | 6.0 | 6.0 |
| | Ethylhexyl Stearate (TEGOSOFT® OS) | 5.0 | 5.0 | 5.0 |
| | Mineral Oil | 4.0 | 4.0 | 4.0 |
| | Ceramide NP | 0.1 | | |
| | Ceramid NP / TEGIN® M Pellets aus Bsp.1 | | 0.6 | |
| | Ceramid NP /TEGO® Alkanol 1618 aus Bsp.1 | | | 0.6 |
| B | Glycerin | 3.0 | 3.0 | 3.0 |
| | Water | 75.9 | 75.9 | 75.9 |
| Z | Preservative, Perfume | q.s. | q.s. | q.s. |
| **Erforderliche Prozesstemperatur** | | **90 °C** | **75 °C** | **75 °C** |

Während man für die Vergleichsformulierung VF1 eine Prozessierungstemperatur von minimal 90 °C benötigt, um das Ceramid vollständig gelöst in die Formulierung eingearbeitet zu bekommen, erreicht man dasselbe Ergebnis bereits bei 75 °C, wenn Ceramid NP in Form einer erstarrten, wachshaltigen Schmelze eingebracht wird.

Die Formulierungen Skin Repair Cream der Tabelle 3 wurden wie folgt hergestellt:
Phase A und B wurden separat auf die angegeben Temperaturen erhitzt, Phase A zu Phase B unter Rühren hinzugefügt und homogenisiert. Es wurde unter langsamem Rühren zunächst auf 60 °C erkalten lassen, dann Phase C hinzugefügt, kurz homogenisiert, Phase D zugefügt und weiter unter langsamem Rühren erkalten lassen.

**Tabelle 3**

| **Phase** | **Ingredients** | **w/w%** | **w/w%** |
|---|---|---|---|
| | | VF2 | EF3 |
| A | Glyceryl Stearate (TEGIN® 4100 Pellets) | 2.5 | 2.5 |
| | Stearyl Alcohol (TEGO® Alkanol 18) | 1.5 | 1.5 |
| | Stearic Acid | 1.0 | |
| | Ceramide NP | 0.2 | |
| | Ceramid NP / Stearic Acid aus Bsp. 1 | | 1.2 |
| | Cetearyl Ethylhexanoate (TEGOSOFT® liquid) | 9.8 | 9.8 |
| | Ethylhexyl Palmitate (TEGOSOFT® OP) | 5.0 | 5.0 |
| | Caprylic/Capric Triglyceride (TEGOSOFT® CT) | 3.0 | 3.0 |
| | Decyl Cocoate (TEGOSOFT® DC) | 2.0 | 2.0 |
| B | Cetearyl Glucoside (TEGO® Care CG 90) | 1.0 | 1.0 |
| | Glycerin | 3.0 | 3.0 |
| | Water | 70.5 | 70.5 |
| C | Carbomer (TEGO® Carbomer 134) | 0.1 | 0.1 |
| | Cetearyl Ethylhexanoate (TEGOSOFT® liquid) | 0.4 | 0.4 |
| D | Sodium Hydroxide (10 % in water) | q.s. | q.s. |
| Z | Preservative, Perfume | q.s. | q.s. |
| Erforderliche **Prozesstemperatur** | | **90 °C** | 75 **°C** |

Während man für die Vergleichsformulierung VF2 eine Prozessierungstemperatur von minimal 90 °C benötigt, um das Ceramid vollständig gelöst in die Formulierung eingearbeitet zu bekommen, erreicht man dasselbe Ergebnis bereits bei 75 °C, wenn Ceramid NP in Form einer erstarrten, wachshaltigen Schmelze eingebracht wird.

Die Formulierungen Natural O/W Lotion der Tabelle 4 wurden wie folgt hergestellt:
Phase A und B wurden separat auf die angegeben Temperaturen erhitzt, Phase A zu Phase B unter Rühren hinzugefügt und homogenisiert. Es wurde unter langsamem Rühren zunächst auf 40 °C erkalten lassen, dann Phase C hinzugefügt, kurz homogenisiert, Phase D und Phase E zugefügt, auf pH 5,2 eingestellt und weiter unter langsamem Rühren erkalten lassen.

**Tabelle 4**

| **Phase** | **Ingredients** | **w/w%** | **w/w%** |
|---|---|---|---|
| | | VF3 | EF4 |
| A | Polyglyceryl-3 Dicitrate/Stearate (TEGO® Care PSC 3) | 3.0 | 2.0 |
| | Caprylic/Capric Triglyceride (TEGOSOFT® CT) | 7.0 | 7.0 |
| | Isopropyl Palmitate (TEGOSOFT® P) | 3.0 | 3.0 |
| | Cetyl Ricinoleate (TEGOSOFT® CR) | 2.0 | 2.0 |
| | Persea Gratissima (Avocado) Oil | 5.0 | 5.0 |
| | Ceramide NP | 0.2 | |
| | Ceramide NP / TEGO® Care PSC 3 aus Bsp. 1 | | 1.2 |
| B | Water | 75.1 | 75.1 |
| | Glycerin | 3.0 | 3.0 |
| C | Keltrol CG-SFT (Xanthan Gum) | 0.5 | 0.5 |
| D | Sodium Hydroxide (10 %) | 0.2 | 0.2 |
| E | Rokonsal BSB-N | 1.0 | 1.0 |
| Z | Perfume | q.s. | q.s. |
| **Erforderliche Prozesstemperatur** | | **90 °C** | **75 °C** |

Während man für die Vergleichsformulierung VF3 eine Prozessierungstemperatur von minimal 90 °C benötigt, um das Ceramid vollständig gelöst in die Formulierung eingearbeitet zu bekommen, erreicht man dasselbe Ergebnis bereits bei 75 °C, wenn Ceramid NP in Form einer erstarrten, wachshaltigen Schmelze eingebracht wird.

### Beispiel 4: Ein-Topf-Verfahren

Die Formulierungen O/W Face Cream im Ein-Topf-Verfahren der Tabelle 5 wurden wie folgt hergestellt:
Wasser und Glycerin wurden separat auf die angegeben Temperaturen erhitzt, die restlichen Inhaltsstoffe nach und nach unter Halten der Temperatur und Rühren hinzugefügt und homogenisiert. Es wurde unter langsamem Rühren erkalten lassen.

**Tabelle 5**

| **Phase** | **Ingredients** | **w/w%** | **w/w%** |
|---|---|---|---|
| | | VF4 | EF5 |
| A | Water | 75.9 | 75.9 |
| | Glycerin | 3.0 | 3.0 |
| | Polyglyceryl-3 Methylglucose Distearate (TEGO® Care 450) | 3.0 | 3.0 |
| | Glyceryl Stearate (TEGIN® M Pellets) | 2.0 | 1.5 |
| | Cetearyl Alcohol (TEGO® Alkanol 1618) | 1.0 | 1.0 |
| | Caprylic/Capric Triglyceride (TEGOSOFT® CT) | 6.0 | 5.0 |
| | Ethylhexyl Stearate (TEGOSOFT® OS) | 5.0 | 5.0 |
| | Mineral Oil | 4.0 | 4.0 |
| | Ceramide NP | 0.1 | |
| | Ceramid NP / TEGIN® M aus Bsp.1 | | 0.6 |
| | Preservative, Perfume | q.s. | q.s. |
| **Erforderliche Prozesstemperatur** | | **90 °C** | **75 °C** |
| Kommentar: | | Ungelöstes Ceramid NP | Ceramid NP vollständig gelöst |

Während in der Vergleichsformulierung VF bei einer Prozessierungstemperatur von 90 °C immer noch ungelöste Ceramid NP Kristalle vorliegen, so sind durch Einsatz von Ceramid NP in Form einer erstarrten, wachshaltigen Schmelze mit TEGIN ® M Pellets Formulierungen mit vollständig gelöstem Ceramid bereits bei einer Prozesstemperatur von 75 °C erhältlich.
Eine Ein-Topf-Prozessierung ist nur wegen der erstarrten Schmelzen möglich.

## Patentansprüche

1. Verfahren zu Herstellung einer ceramidhaltigen Formulierung umfassend die Verfahrensschritte
A) Aufschmelzen einer Mischung enthaltend mindestens ein Wachs und mindestens ein Ceramid,
B) Abkühlen der aufgeschmolzenen Mischung unter Erhalt einer erstarrten Schmelze enthaltend das mindestens eine Wachs und das mindestens eine Ceramid,
C) Einarbeiten der erstarrten Schmelze in eine ölhaltige Formulierung.

2. Verfahren gemäß Anspruch 1, **dadurch gekennzeichnet, dass** das Wachs ausgewählt ist aus der Gruppe bestehend aus natürliche Wachse pflanzlichen oder tierischen Ursprungs, Mineralwachse, Fette, Fettalkohole, Fettsäure, Fettsäureester, Ether von Fettalkoholen, Ester aus Polyethylenoxid und Fettsäuren, Ether aus Polyethylenoxid und Fettalkoholen und partielle Glycerinester.

3. Verfahren gemäß Anspruch 1 oder 2, **dadurch gekennzeichnet, dass** das Ceramid ausgewählt ist aus der Gruppe umfassend Ceramid NP, Ceramid AP, Ceramid EOP, Ceramid NDS , Ceramid ADS, Ceramid EODS, Ceramid NS, Ceramid AS, Ceramid EOS, Ceramid NH, Ceramid AH und Ceramid EOH.

4. Verfahren gemäß mindestens einem der vorherigen Ansprüche, **dadurch gekennzeichnet, dass** das Gewichtsverhältnis von Wachs zu Ceramid in Verfahrensschritt A) 50 : 1 bis 1 : 1, bevorzugt 20 : 1 bis 2 : 1, besonders bevorzugt 9 : 1 bis 4 : 1,
beträgt.

5. Verfahren gemäß mindestens einem der vorherigen Ansprüche, **dadurch gekennzeichnet, dass** die Mischung in Verfahrensschritt A) und die erstarrte Schmelze in Verfahrensschritt B) bezogen auf die Gesamtmischung beziehungsweise Gesamtschmelze 70 Gew.-% bis 100 Gew.-%, bevorzugt 81 Gew.-% bis 99 Gew.-%, besonders bevorzugt 95
Gew.-% bis 98 Gew.-%, das mindestens eine Wachs und das mindestens eine Ceramid enthalten.

6. Verfahren gemäß mindestens einem der vorherigen Ansprüche, **dadurch gekennzeichnet, dass** die Mischung in Verfahrensschritt A)
bei einer Temperatur von 80 °C bis 105 °C, bevorzugt von 85 °C bis 99 °C, besonders bevorzugt von 87 °C bis 92 °C,
aufgeschmolzen wird.

7. Verfahren gemäß mindestens einem der vorherigen Ansprüche, **dadurch gekennzeichnet, dass** die ölhaltige Formulierung in Verfahrensschritt C) eine Emulsion ist und die Verfahrensschritte umfasst
C1) Erwärmen einer Öl-Phase enthaltend die erstarrte Schmelze,
C2) Erwärmen einer wässrigen Phase
C3) Homogenisieren der miteinander vereinigten Öl-Phase und wässrigen Phase.

8. Verfahren gemäß Anspruch 7, **dadurch gekennzeichnet, dass** in Verfahrensschritt C1) und C2)
auf eine Temperatur von 60 °C bis 89 °C, bevorzugt von 65 °C bis 79 °C, besonders bevorzugt von 69 °C bis 76 °C,
erwärmt wird.

9. Verfahren gemäß Anspruch 7 oder 8, **dadurch gekennzeichnet, dass** in der Öl-Phase mindestens ein Öl und mindestens ein Emulgator, insbesondere ein Öl-in-Wasser-Emulgator enthalten sind.

10. Verfahren gemäß mindestens einem der Ansprüche 7 bis 9, **dadurch gekennzeichnet, dass** das Gewichtsverhältnis von Öl-Phase zu wässriger Phase 1 : 100 bis 1: 1, bevorzugt 1: 50 bis 1 : 1,5, besonders bevorzugt 1 : 10 bis 1 : 2,
beträgt.

11. Erstarrte Schmelze einer Mischung enthaltend mindestens ein Wachs und mindestens ein Ceramid, **dadurch gekennzeichnet, dass** bezogen auf die gesamte erstarrte Schmelze Wachs und Ceramid in einer Menge von mindestens 80 Gew.-% in ihr enthalten sind.

12. Erstarrte Schmelze gemäß Anspruch 11, **dadurch gekennzeichnet, dass** bezogen auf die gesamte erstarrte Schmelze Wachs und Ceramid in einer Menge von mindestens 90 Gew.-%, besonders bevorzugt mindestens 98 Gew.-% in ihr enthalten sind.

13. Erstarrte Schmelze gemäß Anspruch 11 oder 12, **dadurch gekennzeichnet, dass** das Ceramid bezogen auf die gesamte erstarrte Schmelze in einer Menge von 2 Gew.-% bis 19 Gew.-%, bevorzugt von 5 Gew.-% bis 18 Gew.-%, besonders bevorzugt von 12 Gew.-% bis 17 Gew.-% enthalten ist.

## Claims

1. Method for preparing a ceramide-containing formulation comprising the method steps of
A) melting a mixture comprising at least one wax and at least one ceramide,
B) cooling the molten mixture to obtain a solidified melt comprising the at least one wax and the at least one ceramide,
C) incorporating the solidified melt into an oil-containing formulation.

2. Method according to Claim 1, **characterized in that** the wax is selected from the group consisting of natural waxes of plant or animal origin, mineral waxes, fats, fatty alcohols, fatty acids, fatty esters, ethers of fatty alcohols, esters of polyethylene oxide and fatty acids, ethers of polyethylene oxide and fatty alcohols and partial glycerol esters.

3. Method according to Claim 1 or 2, **characterized in that** the ceramide is selected from the group comprising Ceramide NP, Ceramide AP, Ceramide EOP, Ceramide NDS, Ceramide ADS, Ceramide EODS, Ceramide NS, Ceramide AS, Ceramide EOS, Ceramide NH, Ceramide AH and Ceramide EOH.

4. Method according to at least one of the preceding claims, **characterized in that** the ratio by weight of wax to ceramide in method step A) is from 50 : 1 to 1 : 1, preferably from 20 : 1 to 2 : 1, particularly preferably from 9 : 1 to 4 : 1.

5. Method according to at least one of the preceding claims, **characterized in that** the mixture in method step A) and the solidified melt in method step B), based on the total mixture and total melt respectively,
comprise 70% by weight to 100% by weight, preferably 81% by weight to 99% by weight, particularly preferably 95% by weight to 98% by weight, of the at least one wax and the at least one ceramide.

6. Method according to at least one of the preceding claims, **characterized in that** the mixture in method step A) is melted
at a temperature of 80°C to 105°C, preferably 85°C to 99°C, particularly preferably 87°C to 92°C.

7. Method according to at least one of the preceding claims, **characterized in that** the oil-containing formulation in method step C) is an emulsion and the method steps comprise
C1) heating an oil phase comprising the solidified melt,
C2) heating an aqueous phase
C3) homogenizing the combined oil phase and aqueous phase with each other.

8. Method according to Claim 7, **characterized in that** in method step C1) and C2)
the phases are heated to a temperature of 60°C to 89°C, preferably 65°C to 79°C, particularly preferably 69°C to 76°C.

9. Method according to Claim 7 or 8, **characterized in that** at least one oil and at least one emulsifier, in particular an oil-in-water emulsifer, are present in the oil phase.

10. Method according to at least one of Claims 7 to 9, **characterized in that**
the ratio by weight of oil phase to aqueous phase is from 1 : 100 to 1 : 1, preferably from 1 : 50 to 1 : 1.5, particularly preferably from 1 : 10 to 1 : 2.

11. Solidified melt of a mixture comprising at least one wax and at least one ceramide, **characterized in that**, based on the total solidified melt, wax and ceramide are present therein in an amount of at least 80% by weight.

12. Solidified melt according to Claim 11, **characterized in that**, based on the total solidified melt, wax and ceramide are present therein in an amount of at least 90% by weight, particularly preferably at least 98% by weight.

13. Solidified melt according to Claim 11 or 12, **characterized in that** the ceramide, based on the total solidified melt, is present in an amount of 2% by weight to 19% by weight, preferably 5% by weight to 18% by weight, particularly preferably 12% by weight to 17% by weight.

## Revendications

1. Procédé de fabrication d'une formulation contenant un céramide, comprenant les étapes de procédé suivantes :
A) la fusion d'un mélange contenant au moins une cire et au moins un céramide,
B) le refroidissement du mélange fondu pour obtenir une masse fondue solidifiée contenant ladite au moins une cire et ledit au moins un céramide,
C) l'incorporation de la masse fondue solidifiée dans une formulation huileuse.

2. Procédé selon la revendication 1, **caractérisé en ce que** la cire est choisie dans le groupe constitué par les cires naturelles d'origine végétale ou animale, les cires minérales, les graisses, les alcools gras, les acides gras, les esters d'acides gras, les éthers d'alcools gras, les esters de polyoxyde d'éthylène et d'acides gras, les éthers de polyoxyde d'éthylène et d'alcools gras et les esters de glycérine partiels.

3. Procédé selon la revendication 1 ou 2, **caractérisé en ce que** le céramide est choisi dans le groupe comprenant le céramide NP, le céramide AP, le céramide EOP, le céramide NDS, le céramide ADS, le céramide EODS, le céramide NS, le céramide AS, le céramide EOS, le céramide NH, le céramide AH et le céramide EOH.

4. Procédé selon au moins l'une quelconque des revendications précédentes, **caractérisé en ce que** le rapport en poids entre la cire et le céramide dans l'étape de procédé A) est de 50:1 à 1:1, de préférence de 20:1 à 2:1, de manière particulièrement préférée de 9:1 à 4:1.

5. Procédé selon au moins l'une quelconque des revendications précédentes, **caractérisé en ce que** le mélange dans l'étape de procédé A) et la masse fondue solidifiée dans l'étape de procédé B) contiennent, par rapport au mélange total ou à la masse fondue totale, 70 % en poids à 100 % en poids, de préférence 81 % en poids à 99 % en poids, de manière particulièrement préférée 95 % en poids à 98 % en poids, de ladite au moins une cire et dudit au moins un céramide.

6. Procédé selon au moins l'une quelconque des revendications précédentes, **caractérisé en ce que** le mélange dans l'étape de procédé A) est fondu à une température de 80 °C à 105 °C, de préférence de 85 °C à 99 °C, de manière particulièrement préférée de 87 °C à 92 °C.

7. Procédé selon au moins l'une quelconque des revendications précédentes, **caractérisé en ce que** la formulation huileuse dans l'étape de procédé C) est une émulsion et l'étape de procédé comprend :
C1) le chauffage d'une phase huileuse contenant la masse fondue solidifiée,
C2) le chauffage d'une phase aqueuse,
C3) l'homogénéisation de la phase huileuse et de la phase aqueuse rassemblées l'une avec l'autre.

8. Procédé selon la revendication 7, **caractérisé en ce que**, dans les étapes de procédé C1) et C2), un chauffage à une température de 60 °C à 89 °C, de préférence de 65 °C à 79 °C, de manière particulièrement préférée de 69 °C à 76 °C, est effectué.

9. Procédé selon la revendication 7 ou 8, **caractérisé en ce qu'**au moins une huile et au moins un émulsifiant, notamment un émulsifiant huile dans eau, sont contenus dans la phase huileuse.

10. Procédé selon au moins l'une quelconque des revendications 7 à 9, **caractérisé en ce que** le rapport en poids entre la phase huileuse et la phase aqueuse est de 1:100 à 1:1, de préférence 1:50 à 1:1,5, de manière particulièrement préférée 1:10 à 1:2.

11. Masse fondue solidifiée d'un mélange contenant au moins une cire et au moins un céramide, **caractérisée en ce que**, par rapport à la masse fondue solidifiée totale, la cire et le céramide sont contenus dans celle-ci en une quantité d'au moins 80 % en poids.

12. Masse fondue solidifiée selon la revendication 11, **caractérisée en ce que**, par rapport à la masse fondue solidifiée totale, la cire et le céramide sont contenus dans celle-ci en une quantité d'au moins 90 % en poids, de manière particulièrement préférée d'au moins 98 % en poids.

13. Masse fondue solidifiée selon la revendication 11 ou 12, **caractérisée en ce que** le céramide est contenu, par rapport à la masse fondue solidifiée totale, en une quantité de 2 % en poids à 19 % en poids, de préférence de 5 % en poids à 18 % en poids, de manière particulièrement préférée de 12 % en poids à 17 % en poids.
